(19) Europäisches Patentamt
European Patent Office
Office européen des brevets

(11) Publication number: 0 440 851 A1

(12) # EUROPEAN PATENT APPLICATION

(21) Application number: 90102425.7

(51) Int. Cl.$^5$: A61K 31/505

(22) Date of filing: 07.02.90

The title of the invention has been amended (Guidelines for Examination in the EPO, A-III, 7.3).

(43) Date of publication of application:
14.08.91 Bulletin 91/33

(84) Designated Contracting States:
AT BE CH DE DK ES FR GB GR IT LI LU NL SE

(71) Applicant: Bristol-Myers Squibb Company
345 Park Avenue
New York, N.Y. 10154(US)

(72) Inventor: Cott, Jerry
15 Windward Drive
New Fairfield, Connecticut 06812(US)
Inventor: Kurtz, Neil M.
83 Valley Forge Road
Weston, Connecticut 06883(US)
Inventor: Robinson, Donald S.
7 Canterbury Way
North Haven, Connecticut 06473(US)

(74) Representative: Kinzebach, Werner, Dr. et al
Patentanwälte Reitstötter, Kinzebach und Partner Sternwartstrasse 4 Postfach 86 06 49
W-8000 München 86(DE)

(54) **Use of buspirone for the treatment of depressions.**

(57) Buspirone and its pharmaceutically acceptable salts are useful in the clinical improvement of major depression including melancholia.

EP 0 440 851 A1

# THE USE OF BUSPIRONE FOR THE TREATMENT OF MELANCHOLIC DEPRESSION

## FIELD OF THE INVENTION

This invention is concerned with the use of the pyrimidine compound 8-[4-[4-(2-pyrimidinyl)-1-piperazinyl]butyl]-8-azaspiro[4.5]decane-7,9-dione or a pharmaceutically acceptable acid addition salt thereof.

## BACKGROUND OF THE INVENTION

The pyrimidine compound with which the present invention is concerned has the following structural formula

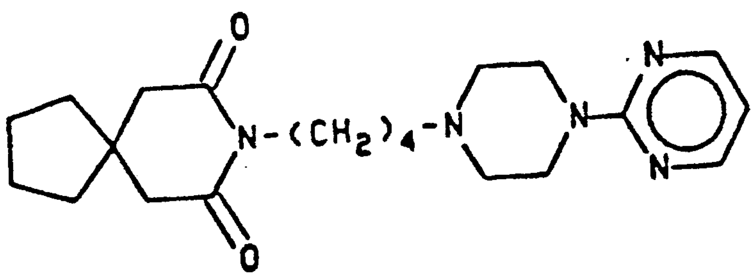

and is known as buspirone. The hydrochloride salt has been referred to in the prior art as MJ 9022-1 and as buspirone hydrochloride. Other acid addition salts thereof are named by combining "buspirone" with the appropriate word to define the acid from which it is prepared as in "buspirone hydrochloride". The latter is the United States Adopted Name (USAN); refer to J. American Med. Assoc. 225, 520 (1973).

The synthesis of the compound and the disclosure of its psychotropic properties are described in the following patents and publications.

1. Y. H. Wu, et al., J. Med. Chem., 15,477 (1972).
2. Y. H. Wu, et al., U. S. Pat. No. 3,717,634 which issued February 20, 1973.
3. L.E. Allen et al., Arzneium. Forsch., 24, No. 6, 917-922 (1974).
4. G. L. Sathananthan, et al., Current Therapeutic Research, 18/5, 701-705 (1975).
5. Y. H. Wu, et al., U. S. Pat. No. 3,976,776, issued August 24, 1976.

The following patent references disclose and claim additional uses that relate to buspirone's pharmacological effects on the central nervous system.

6. The use of buspirone hydrochloride as a novel antianxiety agent for the treatment of neurotic patients is described in G. P. Casten, et al., U. S. Patent No. 4,182,763, issued January 9, 1980.
7. Allen, et al., disclose the use of buspirone in treating extrapyramidal motor disorders in U. S. 4,438,119, issued March 20, 1984.
8. Buspirone's use in sexual dysfunction was described by Othmer, et al., in U. S. 4,640,921, issued February 3, 1987.
9. Kurtz, et al., in U. S. 4,634,703, issued January 6, 1987 disclose buspirone's use in treating panic disorders.
10. Alderdice discloses the use of buspirone in the improvement of short term memory in U. S. 4,687,772, issued August 18, 1987.
11. U. S. 4,777,173 of Shrotriya and Casten, issued October 11, 1988 discloses and claims the use of buspirone in treating alcohol abuse.

There have also been several reports that buspirone has potential for use as a mixed anxiolytic-antidepressant agent (see, for example, Goldberg, et al., J. Clin. Psychiatry, 43/12 Part 2, pp 87-91 (1982)).

Reports of anxiolytic agents demonstrating some antidepressant properties are known, e.g. alprazolam, a benzodiazepine anxiolytic agent with some antidepressant properties. This type of psychotropic profile has been considered to be of value since anxious patients often present with some symptoms of accompanying depression.

It is to be appreciated however that there are different types of depression, ranging from normal degrees of depressed mood, which is universally experienced, to major depressive illness, which is a

condition of significant morbidity and mortality. The increasing severity of the differing types of depression, going from minor, or secondary, forms with partial or milder forms of depression to a major, more severe, primary depressive disorder is consonant with the increasing suffering and misery experienced by such patients and their families. Treatment of depression is complicated unfortunately as depressive illness is not a single entity but a heterogeneous group of disorders, comprised of several subtypes. It is important to realize that these subtypes involve different patient populations and may respond differently to antidepressant treatments. This is evidenced by the observation that about one-third of the patients in a typical treatment population are non-responders or respond only partially. Efforts have continued for years to improve differential diagnosis of the various subtypes of depression so that the most appropriate treatment may be employed. This is particularly important in view of the public health costs of depression and variability of response to treatment.

Adding further complexity to the selection of appropriate antidepressant treatment are differences in standardized diagnostic terminology. For example, the more severe major depressive disorder subtype, melancholia (DSM-III), has been variously classified as: "major affective disorder", "endogenous depression", typical depression", "melancholic depression", "major depressive disorder", "primary depressive disorder" and "depressed phase of affective psychoses". This subtype of depressive illness is characterized by severe manifestations of depression, such as masked retardation or agitation, guilt, early morning awakening, diurnal variation of mood (worse in morning), loss of appetite. Response to electric convulsion therapy and the tricyclic-class of antidepressant drugs is more predictably positive in this patient population.

A different severe primary depressive disorder subtype is classified as "atypical depression". The depressed patients comprising this subtype of depressive illness may be characterized by anxiety, phobic and histrionic symptoms, extreme sensitivity to rejection, emotional overreactivity, being energetic and highly active when not depressed, but suffering fatigue, initial insomnia and reversed diurnal variation (mood worse at night) when depressed. This class of patient may sometimes score low on "depression" psychometric instruments, since its members do not display guilt, delusional ideas, severe weight loss, or suicidal intent and are not usually hospitalized for treatment. Because these patients respond poorly to electric convulsion therapy and standard antidepressant drug treatment, they present real difficulties and suffer much distress. The current treatment of choice employed for "atypical" depressives is administration of monoamine oxidase inhibitors (MAOI's), an older class of drugs that have more restricted usage due their inherent side-effects, not the least of which is the considerable risk due to food and drug incompatabilities with MAOI adminstration. All exposure to indirect acting sympathomimetic amines, particularly tyramine (found in red wine, aged cheeses, aged proteins, etc.) must be avoided in MAOI-treated patients. Many common over-the counter medicines must also be avoided including almost all cold medicines (nasal sprays as well), diet pills, antihistamines, some suppositories and so forth.

Melancholic depression, as well as other primary or major depressive disorders such as psychotic depression or atypical depression may be distinguished from minor depressive disorders and dysthymic states where anxiety with depressed mood may be prominent. These latter tend to be less severe depressive disorders. Also distinguishing these groups of patients is that different treatments are employed for each. Treatments standard for the primary depressive disorders such as tricyclic antidepressant agents, MAO inhibitors and electric convulsive shock, are less apt to be used to treat patients suffering from neurotic disorders with secondary depressive symptoms. These patients are usually treated by such treatment modalities as psychotherapy and/or antianxiety drug therapy. Similarly, treatment appropriate for mixed anxiety-depressive states is not usually effective in relieving the core depression symptoms of primary depressive disorders. While these observations may be used as an empirical guide in selecting an appropriate drug treatment, there is of course no way to predict success of drug therapy beforehand in each of these subtypes.

A pertinent consideration of prior art concerns gepirone, a novel anxiolytic agent with structural and pharmacological similarities to buspirone. Gepirone was disclosed as being useful in certain primary depressive disorders, including melancholic and atypical depression in U.S. 4,771,053, issued September 13, 1988, to Cott, et al. In contrast, until the recent discovery marking the present invention, buspirone was not known to be particularly effective against primary depression, particularly in patients with melancholia where the drug had no effect. (cf: Schweizer, et al., Psychopharmacol. Bull., 22: 183-185 (1986).

The unexpected finding comprising the present invention is that buspirone is effective in treating severely affected patients suffering from primary depressive disorders, particularly major (endogenous) depression with melancholia. Prior teachings of the art suggested that buspirone was not useful in treating the more severe primary depressive illnesses, especially melancholic depression. The pharmacologic profile of buspirone as an anxiolytic agent with antidepressant properties indicated that buspirone could have usefulness in treating the minor, or secondary depression-anxiety subtype of patient. Drugs useful in

treating secondary depression subtypes are not usually employed in treating primary depressive illnesses. In spite of continuing antidepressant drug development activity of the past twenty years, no anxiolytic agent with antidepressant properties has been approved by the FDA for effective treatment of severe primary depressive illnesses.

In summary, buspirone has demonstrated in double-blind, placebo-controlled studies that it is useful in the treatment of certain primary depressive disorders, particularly for more severely affected patients such as those with major depression of the melancholic subtype. It is now appreciated by those skilled in the art that depressive illness is comprised of distinguishable disease states with differently defined patient populations and drug responses. It is further appreciated that agents that are effective in treating one subtype of depression may be ineffective against other of the subtypes; i.e., it is not obvious beforehand if an agent will effectively alleviate a primary depressive disorder of any one clinical subtype. There existed nothing in the prior art that taught buspirone would be useful in alleviation of major depression with melancholia, as well as nonmelancholic depression and atypical depression.

## SUMMARY OF THE INVENTION

The method of the present invention is intended for the alleviation of primary depressive disorders, particularly major depression with melancholia. The method essentially involves administration of buspirone, or a pharmaceutically acceptable acid addition salt thereof, to a human in need of such treatment. For use in the instant method oral administration of buspirone hydrochloride from about 15 to 90 mg per day in divided doses is anticipated as being the preferred dosage regimen.

### Brief Description of the Figure

Figures 1 and 2 graphically demonstrate the time course relationship of eight-week treatment periods of major depression, melancholic (Table 1) and non-melancholic (Table 2), with buspirone. The Hamilton-Depression (Ham-D) psychometric instrument was used to determine individual patient scores at baseline and during the course of the study. Mean patient scores are represented in the tables as a change from the baseline score. A decrease in Ham-D score indicates symptomatic improvement of the patient.

The dotted line represents mean patient Ham-D scores for the patient group being administered placebo medication under double-blind conditions. The buspirone-medicated group is represented by the solid line. The symbol N represents the number of patients whose individual scores were used to determine the mean patient score. Statistical significance is denoted by an asterisk where p-values are less than 0.05 or by a double asterisk where p-valves are less than 0.01.

### Detailed Description of the Invention

Primary depressive illnesses are best classified clinically by using the diagnostic criteria set forth in DSM III (American Psychiatric Association: Diagnostic and Statistical Manual of Mental Disorders, Third Edition. Washington, D.C. APA, 1980. Patients were selected for study using DSM III criteria for Major Depressive Disorder (melancholic or non-melancholic) having a severity of at least 18 on the Hamilton depression scale (Ham-D). The patients consisted of both men and women between the ages of 18 and 89. They were treated for a period of 8 weeks starting at 5 mg t.i.d. with dose increments up to 30 mg daily by day 5 and subsequent adjustments up to a maximum of about 90 mg per day given in divided doses for up to 8 weeks, depending on tolerability and clinical indication. Weekly ratings were made and included the Hamilton Depression Scale (Ham-D) . Safety analyses consisted of weekly monitoring of vital signs and electrocardiograms and biweekly laboratory chemistries. Any other adverse reactions reported by the patient were recorded at each visit. There was a significant overall reduction of Ham-D scores for the entire patient group when weekly efficacy scores were compared to baseline. The mean Ham-D scores for all patient are shown in figures 1 and 2. The patients meeting diagnostic criteria for melancholic depression (according to DSM III) were found to show a favorable response, i.e., their depression scores improved to a similar extent as the non-melancholic patients (compare figures 1 and 2).

These clinical findings demonstrate that buspirone potently alleviates symptoms of severe major depressive illness in clinically relevant populations. Specific use of Hamilton-D psychometric instrument with its rating scale is well known to one skilled in the art and is commonly used to give an indication of the status and severity of clinical depression.

Figures 1 and 2 show the time course relationship of the mean patient Hamilton-D scores. The Hamilton-D score was obtained for each patient at each study week by summing the numerical values

assigned to each Hamilton-D symptom item according to severity and/or frequency being experienced. The higher the patient score the greater the degree of illness.

The study was of double-blind design, that is, neither the patient nor investigator knew whether the patient was receiving active or placebo medication. Over the course of the eight week medication period only the buspirone-treated patient group demonstrated improvement, i.e., significantly lowered Ham-D scores.

In other, open-label studies; buspirone has been demonstrated to be useful in the treatment of patients diagnosed as suffering from atypical depression. Clinical study of buspirone treatment continues in the atypical depression subgroup of patients with major depression.

In summary it has now been found that buspirone alleviates major depressive illness in some subgroups of patients, specifically including those suffering from major depression of the melancholia subtype. These findings have been demonstrated by analysis of changes in depressive illness symptomatology-related scale items on standard depression psychometric instruments.

Due to these findings, buspirone is currently under study in prospective clinical trials in order to gain approval from the U.S. Food and Drug Administration for the use of buspirone for treatment of major depressive illness.

The process of the present invention then essentially involves administration of buspirone, or a pharmaceutically acceptable acid addition salt thereof, to a human in need of such treatment. Pharmaceutically acceptable acid addition salts of buspirone and methods of pharmaceutical formulation are described in the patent of Wu, et al, U. S. Patent No. 3,976,776 which is incorporated herein in its entirety by reference.

Administration of buspirone according to the present invention may be made by the parenteral, oral or rectal route. The oral route is preferred, however. The clinical dosage range for alleviation of major depressive disorders is expected to be generally higher than for anxiolytic treatment but in most cases on the order of less than about 100 mg per day, more commonly in the 45 to 90 mg range. Since the dosage should be tailored to the individual patient, the usual practice is to commence with a dose of about 5 to 10 mg administered several times per day and then to increase the dose every 2 or 3 days by 5 mg at each dosage time until the desired response is observed or until the patient exhibits side effects. Single daily dosage may be applicable in some instances, but division of the daily dose into 2 or more portions is the preferred practice.

**Claims**

1. The use of buspirone or a pharmaceutically acceptable acid addition salt thereof for preparing a pharmaceutical composition for alleviation of major depressive disorders comprising major depression with melancholia, major non-melancholic depression, and atypical depression.

2. The use of claim 1 wherein major depression with melancholia is the specific major depressive disorder.

3. The use of claim 1 wherein major non-melancholic depression is the specific primary depressive disorder.

4. The use of claim 1 wherein atypical depression is the specific primary depressive disorder.

5. The use of claims 2 or 3 wherein a daily dose of from about 15 mg to 100 mg is employed.

6. The use of claim 4 wherein said daily dose is given in divided doses.

7. The use of claim 4 wherein said daily dose is divided and administered t.i.d.

8. The use of claim 4 wherein buspirone hydrochloride is employed and dosage is by the oral route.

Table 1

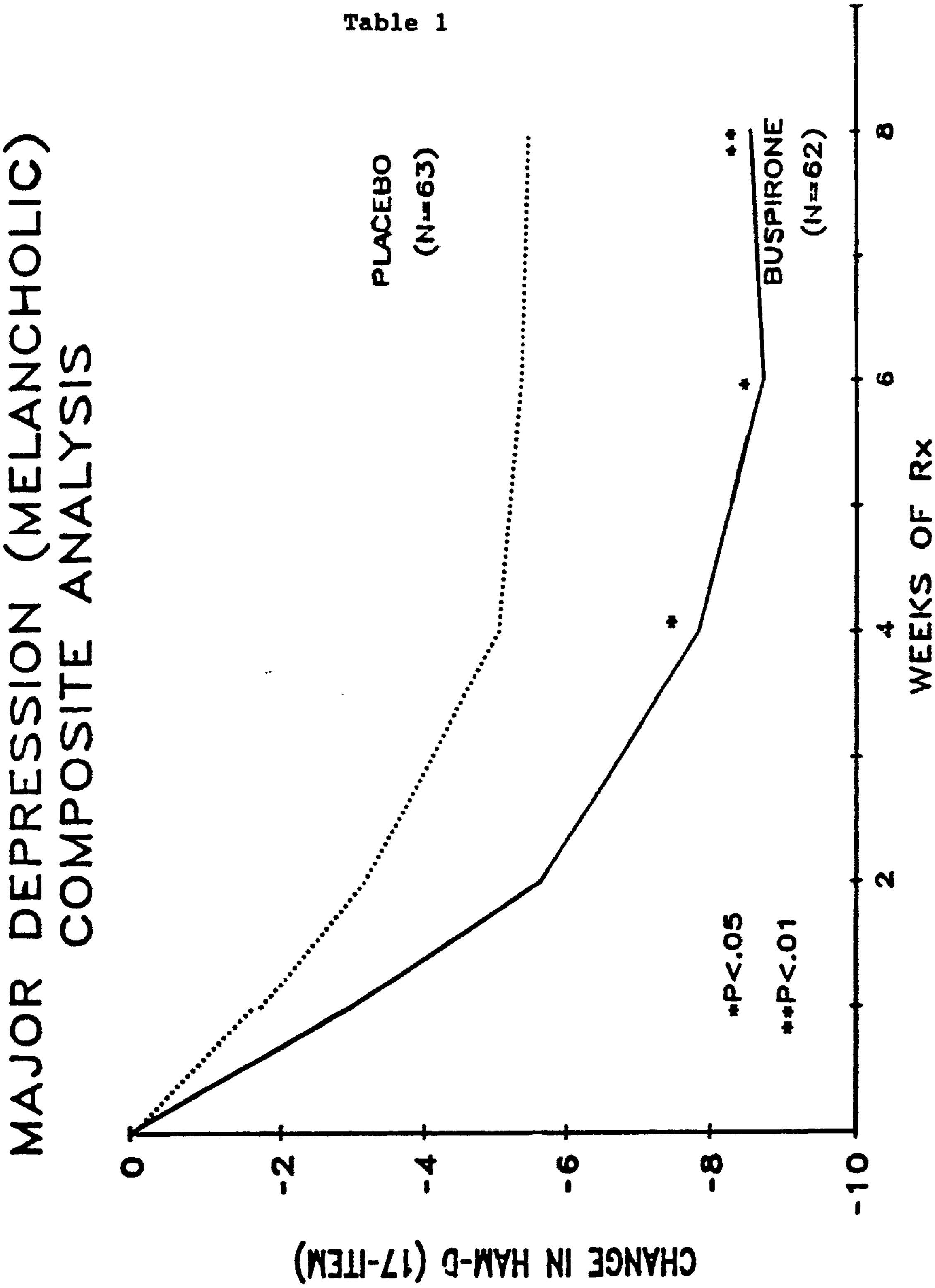
MAJOR DEPRESSION (MELANCHOLIC)
COMPOSITE ANALYSIS
PLACEBO
(N=63)
BUSPIRONE
(N=62)
*P<.05
**P<.01
CHANGE IN HAM-D (17-ITEM)
0
-2
-4
-6
-8
-10
WEEKS OF Rx
2
4
6
8

Table 2

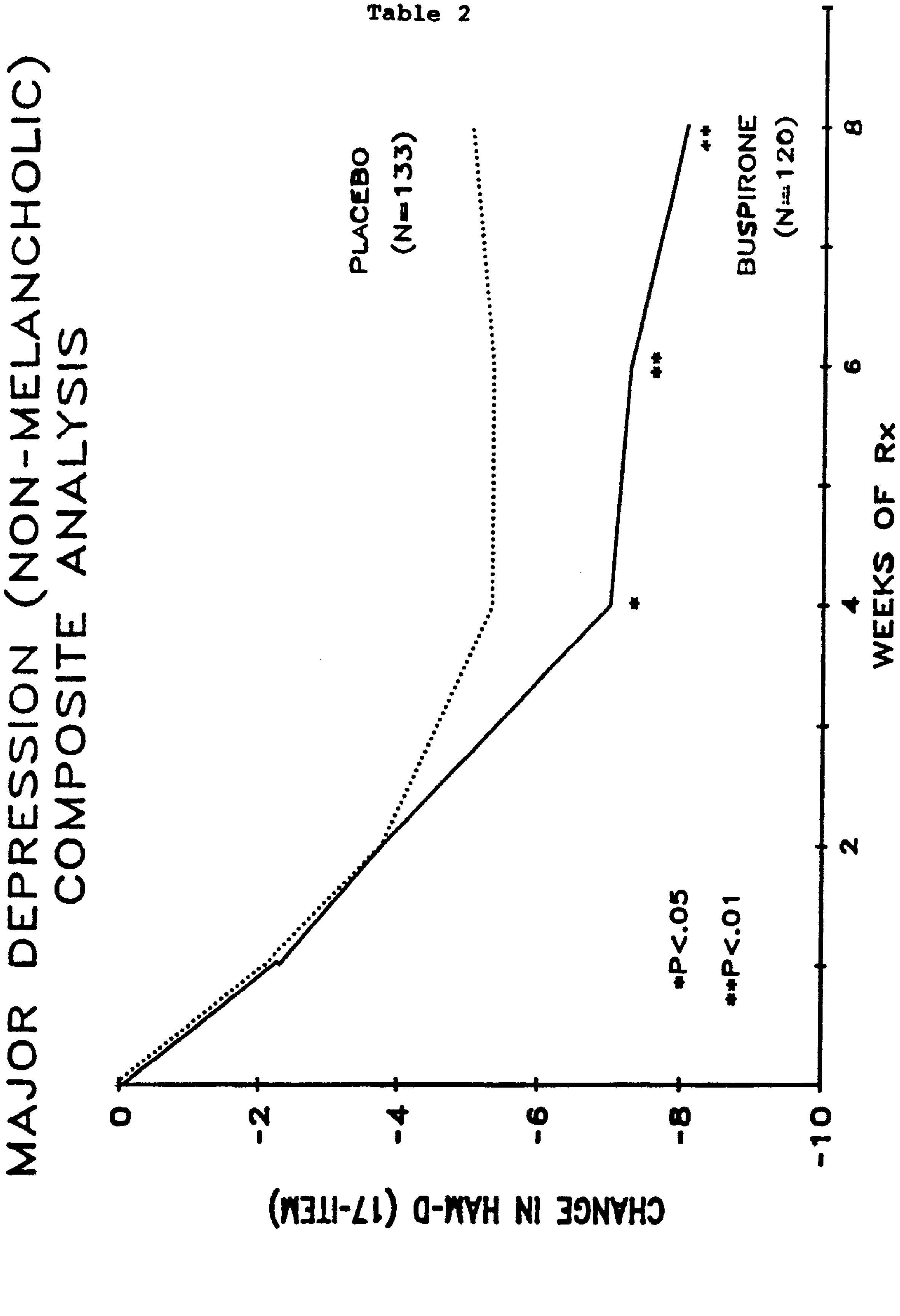
MAJOR DEPRESSION (NON-MELANCHOLIC)
COMPOSITE ANALYSIS
PLACEBO
(N=133)
BUSPIRONE
(N=120)
*P<.05
**P<.01
*
**
**
CHANGE IN HAM-D (17-ITEM)
0
-2
-4
-6
-8
-10
2
4
6
8
WEEKS OF Rx

European Patent Office

## PARTIAL EUROPEAN SEARCH REPORT

which under Rule 45 of the European Patent Convention shall be considered, for the purposes of subsequent proceedings, as the European search report

Application number

EP 90 10 2425

### DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,X | PSYCHOPHARMACOL. BULL. vol. 22, no. 1, 1986, page 183-186; E.E. SCHWEIZER et al.: "Open trial of buspirone in the treatment of major depressive disorder"<br>* Whole article *<br>-- | 1-4 | A 61 K 31/505 |
| X | AM. J. PSYCHIATRY, vol. 136, no. 9, September 1979, pages 1184-1187, American Psyciatric Association; H.L. GOLDBERG et al.: "The comparative efficacy of buspirone and diazepam in the treatment of anxiety"<br>* Whole article *<br>--<br>./. | 1-4 | |

TECHNICAL FIELDS SEARCHED (Int. Cl.4)

A 61 K

### INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-4

Claims searched incompletely:

Claims not searched: 5-8

Reason for the limitation of the search:

Method for treatment of the human or animal body by surgery or therapy (See art. 52(4) of the European Patent Convention)

| Place of search | Date of completion of the search | Examiner |
|---|---|---|
| The Hague | 05-09-1990 | GOETZ |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

European Patent Office

# PARTIAL EUROPEAN SEARCH REPORT

Application number EP 90 10 2425 -2-

| Category | Citation of document with indication, where appropriate, of relevant passages | Relevant to claim | CLASSIFICATION OF THE APPLICATION (Int. Cl.4) |
|---|---|---|---|
| X | PSYCHOPATHOLOGY, vol. 22 (suppl.), 1989, pages 27-36, S. Karger AG, Basel, CH; D.S. ROBINSON et al.: "Serotonergic anxiolytics and treatment of depression"<br>* Whole article *<br>-- | 1-4 | |
| X | EUROPEAN JOURNAL OF PHARMACOLOGY, vol. 134, 1987, pages 265-274, Elsevier Science Publishers B.V.; G.A. KENNETT et al.: "Antidepressant-like action of 5-$HT_{1A}$ agonists and conventional antidepressants in an animal model of depression"<br>* Whole article *<br>-------- | 1-4 | TECHNICAL FIELDS SEARCHED (Int Cl.4) |